# EUROPÄISCHE PATENTANMELDUNG

(11) **EP 0 698 661 A2**
(43) Veröffentlichungstag der Anmeldung: **28.02.1996**
(21) Anmeldenummer: 95112117.7
(22) Anmeldetag: 02.08.1995
(51) Int. Cl.: C11D 3/39

(54) **Aktivatoren für anorganische Peroxoverbindungen und sie enthaltende Mittel**

(30) Priorität: 25.08.1994 DE 4430071
(71) Anmelder: Degussa Aktiengesellschaft, D-60311 Frankfurt (DE)
(72) Erfinder: Dankowski, Manfred, Dr., D-63776 Mömbris (DE); DelGrosso, Michael, Dr., D-63579 Freigericht (DE); Dorfer, Astrid, Dr., D-63584 Gründau (DE)

(57) **Zusammenfassung**

Zur Aktivierung anorganischer Peroxoverbindungen in Wasch-, Bleich-, Reinigungs- und Desinfektionsmitteln finden O- und N-Acylverbindungen Verwendung.

Gefunden wurde, daß Verbindungen der Formel (I) 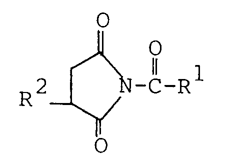
worin R¹ für eine C₆- bis C₁₀-Alkylgruppe und R² für Wasserstoff oder ausgewählte Substituenten steht, hochwirksame Aktivatoren für wäßrige Bleich-, Wasch-, Reinigungs- und Desinfektionsflotten sind. Ein besonders bevorzugter Aktivator ist N-n-Nonanoylsuccinimid. Die Erfindung betrifft auch Bleich-, Wasch-, Reinigungs- und Desinfektionsmittel, enthaltend eine anorganische Peroxoverbindung und einen erfindungsgemäßen Aktivator.

## Beschreibung

Die Erfindung richtet sich auf die Verwendung von N-Alkanoylverbindungen als Aktivatoren für Wasserstoffperoxid und dieses in wäßriger Phase freisetzende anorganische Peroxoverbindungen, wobei die Aktivierung in der Bildung einer organischen Percarbonsäure besteht. Ein weiterer Gegenstand richtet sich auf erfindungsgemäße Aktivatoren und anorganische Peroxoverbindungen enthaltende Wasch-, Bleich-, Reinigungs- und Desinfektionsmittel.

Anorganische Persauerstoffverbindungen werden als Oxidationsmittel in Bleich-, Wasch-, Reinigungs- und Desinfektionsmitteln eingesetzt, um die Wirkung derartiger Mittel zu verbessern. Als Persauerstoffverbindungen kommen insbesondere Wasserstoffperoxid und solche Stoffe zum Einsatz, welche in wäßriger Lösung Wasserstoffperoxid freisetzen, wie Perborate und Percarbonate. Die Wirkung der anorganischen Persauerstoffverbindungen hängt außer vom pH-Wert maßgeblich von der Temperatur ab. Während bei Temperaturen oberhalb etwa 80 °C eine gute Wirkung erzielt wird, ist bei der Verwendung der genannten anorganischen Persauerstoffverbindungen bei niedrigeren Temperaturen, insbesondere um/unter 60 °C oder um/unter 40 °C, die Mitverwendung sogenannter Aktivatoren erforderlich. Bei den Aktivatoren handelt es sich überwiegend um N-Acyl- oder O-Acylverbindungen. In wäßriger Phase bilden sich aus H₂0₂ oder dieses freisetzenden anorganischen Persauerstoffverbindungen und den Aktivatoren Percarbonsäuren, welche auch im Niedertemperaturbereich eine gute Wasch-, Reinigungs-, Bleich- und Desinfektionswirkung entfalten.

Unter den N-Acylverbindungen sind zahlreiche Stoffklassen als Aktivatoren vorgeschlagen worden, darunter: N,N,N',N'-Tetraacetyl-ethylendiamin (TAED), N,N,N',N'-Tetraacetylglykoluril (TAGU), N-(C₁-bis C₄)- bzw. N-(C₆-bis C₁₀)- Alkanoylhydantoine (DE-C 19 49 561 bzw. US 4,412,934), N,N'-(C₁-bis C₈-)-Alkanoyl-2,5-diketopiperazine (DE-A 20 38 106) und N-(C₁-bis C₄-)-Alkanoylsuccinimid (DE-C 19 49 561 und GB-B 855735). Trotz dieser Vielfalt konnte sich im Markt von den N-Acylverbindungen im wesentlichen nur TAED durchsetzen.

Aus dem US-Patent 4,412,934 sind Bleichmittelzusammensetzungen bekannt, welche als Aktivator Stoffe der Formel R-CO-L enthalten, wobei R eine Alkylgruppe mit 5 bis 18 C-Atomen ist, deren längste Kette R-CO 6 bis 10 C-Atome aufweist, und die konjugierte Säure der Abgangsgruppe L einen pKₐ-Wert von 6 bis 13 aufweist. Unter den Abgangsgruppen werden viele über Sauerstoff, aber auch einige wenige über Amidstickstoff an R-CO gebundene Gruppen offenbart, darunter der N-Hydantoinylrest, nicht aber der N-Succinylrest. Bevorzugte Abgangsgruppen sind Phenolderivate; der Rest R steht bevorzugt für lineares (C₅-bis C₉)-Alkyl; besonders bevorzugt wird der Aktivator Nonanoyloxybenzolsulfonat (NOBS). NOBS ist ein sehr wirksamer, in handelsüblichen Bleichmittelzusammensetzungen enthaltender Aktivator, deren Bleichwirkung diejenige von TRED übersteigt.

In Anbetracht des wachsenden Bedarfs an Wasch-, Bleich-, Reinigungs- und Desinfektionsmitteln für den Niedertemperaturbereich besteht ein Interesse an weiteren Aktivatoren auf der Basis von N-Acylverbindungen, welche an das Eigenschaftbild von NOBS heranreichen oder dieses übertreffen. Die Aktivatoren sollten nach Möglichkeit aus leicht verfügbaren Rohstoffen zugänglich und gut biologisch abbaubar sein.

Die Aufgabe wird gelöst durch die Verwendung von N-Alkanoylverbindungen der allgemeinen Formel (I), 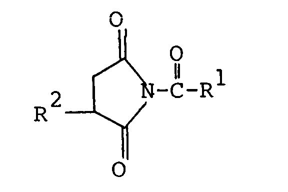
worin R¹ für einen C₆-bis C₁₀-Alkylrest und R² für Wasserstoff, eine HOOC- oder HO₃S-Gruppe oder ein Salz davon, eine C₁-bis C₄-Alkyl- oder eine Hydroxylgruppe steht, als Aktivator für Wasserstoffperoxid und dieses freisetzende anorganische Peroxoverbinderungen in wäßrigen Bleich-, Wasch-, Reinigungs- und Desinfektionsflotten.

Bei der erfindungsgemäßen Verwendung bildet sich in den wäßrigen Flotten, deren pH-Wert auf größer 4 bis 13, vorzugsweise auf 8 bis 11, eingestellt wird, durch Perhydrolyse des Aktivators der Formel (I) eine bleich- und desinfektionswirksame Percarbonsäure mit 7 bis 11 C-Atomen. Besonders wirksam ist die Peroxo-n-nonansäure, weshalb die Verwendung von Aktivatoren der Formel (I) mit R¹ gleich n-Octyl bevorzugt werden. Wegen ihrer leichten Zugänglichkeit werden ferner Aktivatoren mit R² gleich Wasserstoff bevorzugt.

Die erfindungsgemäße Verwendung betrifft die Aktivierung von H₂0₂ und solchen anorganischen Persauerstoffverbindungen, welche in wäßriger Phase Wasserstoffperoxid freisetzen. Hervorzuheben sind Perborate, insbesondere Natriumperborat-monohydrat und Natriumperborat-tetrahydrat, superoxidiertes Natriumperborat und Natriumpercarbonat (2 Na₂CO₃ · 3 H₂0₂). Einsetzbar sind auch Perphosphate, Persilikate und Persulfate. Bei der Aktivierung können auch mehrere anorganische Peroxoverbindungen zugegen sein.

Zur Aktivierung werden pro Äquivalent Aktivsauerstoff des anwesenden und aus den anorganischen Peroxoverbindungen freisetzbaren Wasserstoffperoxid 1 bis 0,05 Mol, vorzugsweise 0,5 bis 0,1 Mol Aktivator der Formel (I) eingesetzt.

Die erfindungsgemäß zu verwendenden Aktivatoren können zur Aktivierung in reiner Form oder mit Hilfsstoffen, wie Granulierhilfsmitteln, Stabilisatoren, pH-regulierenden Stoffen, eingesetzt werden; geeignete Zugabeformen sind Pulver, Pasten, Tabletten, Granulate oder umhüllte Granulate.

Erfindungsgemäß zu verwendende Aktivatoren sind durch übliche Acylierung von Succinimid oder gemäß Formel (I) monosubstituiertem Succinimid, wie Apfelsäureimid, 2-Methylsuccinimid, 2-Carboxysuccinimid oder 2-Sulfosuccinimid, mit dem gewünschten Alkanoylhalogenid mit 7 bis 11 C-Atomen, wie etwa Nonanoylchlorid, erhältlich. Während N-Alkanoyl-succinimid mit 1 bis 5 sowie 12 und 18 C-Atomen im Alkanoylrest beschrieben sind, handelt es sich bei den erfindungsgemäßen Aktivatoren, wie dem besonders bevorzugten Nonanoylsuccinimid, um neue Stoffe.

Je nach Verwendungszweck können die Aktivatoren und anorganischen Persauerstoffverbindungen außer in rein wäßriger Phase auch in wäßrig-organischer Phase zum Einsatz kommen. Ein rein wäßriges Milieu liegt bei den üblichen Wasch-, Bleich- und Reinigungsflotten vor. Ein wäßrigorganisches Milieu kann bei Anwendungen zur Desinfektion sowie bei technischen Oxidationsprozessen zweckmäßig sein. Der pH-Wert des Reaktionsmediums kann zwischen etwa 4 und 13 liegen, vorzugsweise wird aber im alkalischen Bereich, meist bei pH 8 bis 11, gearbeitet, da in diesem Bereich sowohl die in situ-Bildung der organischen Persäure gut abläuft als auch die Stabilität der Peroxoverbindungen befriedigend ist.

Ein weiterer Gegenstand der Erfindung richtet sich auf Bleich-, Wasch-, Reinigungs- und Desinfektionsmittel, welche eine anorganische Peroxoverbindung und einen Aktivator aus der Reihe der erfindungsgemäß verwendbaren N-acylierten Succinimide und N-acylierten monosubstituierten Succinimide der zuvor genannten Formel (I) enthalten. N-n-Nonanoylsuccinimid wird als Aktivator in diesen Mitteln bevorzugt. Als anorganische Peroxoverbindung enthalten die Mittel wiederum die zuvor genannten Stoffe, insbesondere Natriumperborate und Natriumpercarbonat. Pro Äquivalent Aktivsauerstoff aus der oder den anorganischen Peroxoverbindungen enthalten die Mittel 1 bis 0,05 Mol, vorzugsweise 0,5 bis 0,1 Mol Aktivator der Formel (I).

In den Mitteln können ein oder mehrere anorganische Persauerstoffverbindungen sowie ein oder mehrere Aktivatoren, darunter mindestens ein erfindungsgemäßer und bei Bedarf auch handelsübliche oder andere vorbekannte Aktivatoren enthalten sein.

Erfindungsgemäß zu verwendende Aktivatoren und anorganische Persauerstoffverbindungen können mit allen üblichen Bestandteilen von Wasch- und Bleichmitteln kombiniert werden, um zu Wasch- und Bleichmitteln zu gelangen, welche zur Textilbehandlung im Nieder- und Mitteltemperaturbereich, aber auch zur Kochwäsche geeignet sind.

Hauptbestandteile solcher Wasch- und Bleichmittel sind, neben den erwähnten Peroxoverbindungen und Aktivatoren, Gerüstsubstanzen (Builder) und Tenside. Unter den Buildern sind insbesondere Natriumaluminiumsilikate (Zeolithe), Schichtsilikate, kondensierte Phosphate, Alkalisilikate, Alkalicarbonate, komplexierende Aminocarbonsäuren, Polyphosphonsäuren, mehrwertige Hydroxycarbonsäuren sowie Polycarbonsäuren und Salze der genannten Säuren zu nennen. Unter den Tensiden sind insbesondere nichtionische Tenside, wie Fettalkohol- und Alkylphenol-polyethylenglykolether sowie langkettige Alkylglykoside, und anionische Tenside, wie Alkylbenzolsulfonate und Sulfate von Fettalkoholen und Polyethylenglykolmonoethern, hervorzuheben. Weitere Stoffe der Wasch- und Bleichmittel sind Elektrolyte, pH-regulierende Stoffe, Stabilisatoren, Schaumregulatoren, Vergrauungsinhibitoren, optische Aufheller, Enzyme, Avivagemittel. Die in derartigen Mitteln zu verwendenden Stoffe und Einsatzmengen sind dem Fachmann bekannt - eine Übersicht nebst Literatur vermittelt H.G. Hauthal in "Chemie in unserer Zeit" 26 (1992) Nr. 6, 293-303).

Üblicherweise setzen sich erfindungsgemäße Wasch- und Bleichmittel etwa wie folgt zusammen:
- 5 bis 30 Gew.-%,: vorzugsweise 10 bis 25 Gew.-% anionische und/oder nichtionische Tenside,
- 5 bis 60 Gew.-%,: vorzugsweise 20 bis 40 Gew.-% Gerüstsubstanzen aus der Gruppe Natriumaluminiumsilikate, kondensierte Phosphate, Alkalisilikate, Alkalicarbonate, und deren Mischungen,
- 0 bis 20 Gew.-%,: vorzugsweise 1 bis 8 Gew.-% Gerüstsubstanzen aus der Gruppe komplexierender Aminocarbonsäuren, Polyphosphonsäuren, Polycarbonsäuren oder deren Salze sowie deren Mischungen,
- 2 bis 35 Gew.-%,: vorzugsweise 10 bis 25 Gew.-% anorganische Peroxoverbindungen aus der Gruppe Natriumperborate und Natriumpercarbonat,
- 0,2 bis 20 Gew.-%,: vorzugsweise 1 bis 10 Gew.-% erfindungsgemäß zu verwendende N-Alkanoyl-succinimidverbindungen der Formel (I) als Aktivatoren
- ad 100 Gew.-%: übliche Hilfsstoffe und Wasser.

Reine Bleichmittel, wie sie als Zusatzmittel für bleichmittelfreie Waschmittel zur Anwendung gelangen können, setzen sich im allgemeinen wie folgt zusammen:
- 5 bis 50 Gew.-%,: insbesondere 15 bis 35 Gew.-%, anorganische Persauerstoffverbindungen, insbesondere Na-perborat-monohydrat oder -tetrahydrat oder/und Natriumpercarbonat,
- 2 bis 50 Gew.-%,: insbesondere 5 bis 25 Gew.-% erfindungsgemäß zu verwendende N-Alkanoyl-succinimidverbindungen der Formel (I) als Aktivatoren,
- 0 bis 5 Gew.-%,: Peroxidstabilisatoren, wie Wasserglas und Komplexbildner,
- 0 bis 40 Gew.-%,: pH-regulierende Mittel
- ad 100 Gew.-%: andere übliche Hilfsstoffe.

Erfindungsgemäße Reinigungsmittel enthalten im allgemeinen Tenside, Builder, Persauerstoffverbindungen und erfindungsgemäß zu verwendende Aktivatoren; Scheuermittel enthalten zusätzlich abrasiv wirkende Bestandteile.

Erfindungsgemäße Desinfektionsmittel basieren im allgemeinen auf einer Kombination aus anorganischen Peroxoverbindungen und erfindungsgemäß zu verwendenden Aktivatoren sowie Hilfsstoffen aus der Reihe der Stabilisatoren, Tenside, pH-regulierenden Stoffe und, sofern erwünscht, organischen Lösungsmitteln und anderen mikrobioziden Stoffen als die aus den Aktivatoren und Peroxoverbindungen entstehenden Percarbonsäuren.

Es wurde festgestellt, daß die Aktivatorwirkung der erfindungsgemäß zu verwendenden Aktivatoren auf der Basis von N-Acylverbindungen, diejenige vorbekannter N-Acylverbindungen, wie das im Markt eingeführte TAED, in sprunghafter Weise übersteigt. Unerwarteterweise kommt die Aktivatorwirkung auch nicht nur an diejenige des bisher wirksamsten Aktivators NOBS heran, sondern übersteigt diese. Die herausragende Wirkung der Aktivatoren der Formel (I) und insbesondere des N-n-Nonanoylsuccinimids war überraschend, weil im Stand der Technik bereits N-Alkanoylsuccinimide mit wenigen C-Atomen in der Alkanoylgruppe als Aktivatoren beschrieben wurden. Die Wirkung von N-n-Nonanoylsuccinimid ist gegenüber nichterfindungsgemäßem N-Acetylsuccinimid, gemessen an Delta Remissionszunahme (%) nach Waschen im Launder-O-meter unter US-Waschbedingungen bei 30 °C und gewichtsgleicher Dosierung des Aktivators, um den Faktor von etwa 2,3 höher. Aus der nachfolgenden Tabelle geht ferner die Aktivatorwirksamkeit von TAED und NOBS hervor. Die für die Tests eingesetzten Waschmittelzusammensetzungen und die Anwendungskonzentration derselben sind Beispiel 2 zu entnehmen.

**Tabelle 1**

| Delta Remissionszunahme (%) * | | |
|---|---|---|
| Aktivator | Mittelwert ** | Ketchup |
| N-n-Nonanoylsuccinimid | 1,7 | 2,3 |
| N-Acetylsuccinimid | 0,7 | -0,3 |
| TAED | 0,4 | -1,5 |
| NOBS | 1,4 | 1,0 |
| ohne Aktivator | 0,0 | 0,0 |

| | | |
|---|---|---|
| * Delta Remissionszunahme (%) resultiert aus der Differenz der Remissionszunahme bei aktivator- und bleichmittelhaltigem Waschmittel abzüglich Remissionszunahme bei bleichmittel- und aktivatorfreiem Waschmittel. | | |
| ** Mittelwert aus je neun Testanschmutzungen mit Kaffee, Tee, Rotwein, Paprika, Ketchup, Curry. | | |

Aus der vorstehenden Tabelle geht auch die außergewöhnliche Wirkung des erfindungsgemäßen Aktivators gegenüber NOBS bei der Bleiche von Tomaten-Ketchup-Flecken hervor.

Die Persäurefreisetzung aus erfindungsgemäßen Aktivatoren und Natriumperborat erfolgt gegenüber N-Acetyl-succinimid, und gegenüber NOBS verzögert, was im Hinblick auf den Erhalt der Wirksamkeit von im Waschmittel gegebenenfalls enthaltenen Enzymen vorteilhaft sein kann.

### Beispiel 1

### N-Nonanoylsuccinimid

20 g Succinimid wurden in 100 ml Pyridin suspendiert, mit 0,5 g N,N-Dimethylaminopyridin versetzt und bei Eisbadtemperatur 39,2 g Nonansäurechlorid zugetropft. Anschließend rührte man 1 h bei Raumtemperatur nach und versetzte unter Kühlung mit 500 ml 2 N-HCl-Lösung. Die wäßrige Phase wurde mit Essigester extrahiert, die organische Phase mit 2 N-HCl-Lösung gewaschen und getrocknet (Na₂SO₄). Nach Entfernen des Lösungsmittels wurde der Rückstand zweimal aus n-Hexan umkristallisiert. Man erhielt 31,6 g (65 %) N-Nonanoylsuccinimid als farblosen Feststoff.
Schmp: 59 bis 60 °C (n-Hexan).
Die ¹-NMR-spektroskopischen Daten stehen mit der Struktur im Einklang.

### Beispiel 2

Untersuchung der Aktivatorwirkung von Nonanoylsuccinimid im Vergleich zu nicht-erfindungsgemäßen Aktivatoren N-Acetylsuccinimid, TAED und NOBS.
- Waschgerät:: Launder-O-meter
- Waschtemperatur:: 30 °C
- Wasserhärte:: 5 °d
- Waschprogramm:: 500 ml Waschbehälter,
200 ml Waschflotte,
15 min Waschzeit,
3 x 30 sec Spülzeit
- Flottenverhältnis:: 1 : 20
- Dosierung:: 1,35 g/l gleich 0,27 g/Waschgang
eines in USA handelsüblichen bleichmittel- und aktivatorfreien Waschmittels (enthaltend anionische Tenside, Zeolith A, Natriumcitrat, Natriumsulfat, Natriumsilikat, Soda und Enzyme)
je 0,015 g/Waschgang Natriumperboratmonohydrat und Aktivator
- Testgewebe:: Baumwolle
- Testflecken:: Kaffee, wfk 10 K; Tee wfk CFT BC-1;
Paprika wfk 10 N; Curry wfk CFT BC-4;
Rotwein EMPA 114; Tomaten-Ketchup wfk 10 T.

Nach der Wäsche der Testgewebe wurde jeweils die Remissionszunahme gemessen und mit der Remissionszunahme, erhalten mittels aktivator- und bleichmittelfreiem Waschmittel verglichen (=Delta Remission). Die für die Remissionszunahme zugrundegelegten Werte sind Mittelwerte aus neun gleichen Anschmutzungen.

Die Ergebnisse folgen aus der zuvor bereits beschriebenen Tabelle 1.

### Beispiel 3

Persäurefreisetzungen von N-Nonanoylsuccinimid im Vergleich zu jener von N-Acetylsuccinimid.

In Wasser von 30 °C wurden 8 g/l bleichmittel- und aktivatorfreies Waschmittel, 1,5 g/l Natriumperboratmonohydrat und 0,5 g/l Aktivator eingewogen und die zeitabhängige Bildung von Pernonansäure beziehungsweise Peressigsäure bestimmt.

Die folgende Tabelle zeigt die Persäureäquivalente nach der Zeit t bezogen auf 1 Mol Aktivator.

**Tabelle 2**

| Zeit (Min) | Äquivalente Persäure | |
|---|---|---|
| | N-Nonanoylsuccinimid | N-Acetylsuccinimid |
| 2 | 0,54 | 1,0 |
| 6 | 0,81 | 1,0 |
| 10 | 0,87 | 1,0 |
| 15 | 0,89 | 0,99 |
| 20 | 0,90 | 0,98 |
| 30 | 0,92 | 0,95 |

### Beispiel 4

Aktivatorwirkung von N-n-Nonanoylsuccinimid und NOBS bei 30 °C in Gegenwart von Natriumperborat und eines enzymhaltigen Waschmittels:
Gewaschen wurde Baumwoll-Testgewebe mit Blutanschmutzung (wfk CFT CS1). Waschgerät, Waschtemperatur, Waschprogramm und Flottenverhältnis gemäß Beispiel 2; Wasserhärte 14 °d.

Dosierung:
Kompaktwaschmittel: 3,8 g/l = 0,76 g/Waschgang;
Waschmittelrezeptur in g/l Flotte:

| Waschmittelbestandteile in g/l Flotte | |
|---|---|
| Alkylbenzolsulfonate | 0,52 |
| Fettalkoholethoxylate | 0,35 |
| Seife | 0,10 |
| Zeolith A | 1,52 |
| Polycarboxylate | 0,18 |
| Soda | 0,76 |
| Na- und Mg-silikate | 0,24 |
| CMC | 0,06 |
| Hilfsstoffe (insgesamt) | 0,07 |
| | 3,8 g/l |

Enzyme: 0,085 g/l = 0,0171 g/Waschgang Protease (Savinase der Firma Novo)
Bleichmittel: 0,144 g/Waschgang Natriumperborat-monohydrat (SPM)
Aktivator:
a) 0,125 g/Waschgang N-n-Nonanoylsuccinimid
b) 0,144 g/Waschgang NOBS
Die Dosierung der Aktivatoren erfolgte persäuregleich:
Oₐ aus H₂O₂ jeweils 76 mg/l; Oₐ aus Persäure (berechnet) je 33 mg/l.

Nach der Wäsche wurde die Remissionszunahme gemessen:

**Tabelle 3**

| | Remissionszunahme (%) |
|---|---|
| a) N-n-Nonanoylsuccinimid + SPM | 8,9 |
| b) NOBS + SPM | 8,1 |

Der Vergleich zeigt, daß der erfindungsgemäße Aktivator die Wirkung des vorbekannten Aktivators übertrifft.

## Patentansprüche

1. Verwendung von N-Alkanoylverbindungen der allgemeinen Formel (I), 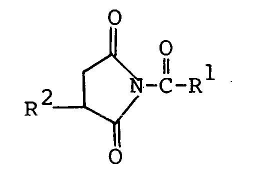 worin R¹ für einen C₆-bis C₁₀-Alkylrest und R² für Wasserstoff, eine HOOC- oder HO₃S-Gruppe oder ein Salz der Säuren, eine C₁-bis C₄-Alkyl- oder eine Hydroxylgruppe steht, als Aktivator für Wasserstoffperoxid und dieses freisetzende anorganische Peroxoverbinderungen in wäßrigen Bleich-, Wasch-, Reinigungs- und Desinfektionsflotten.

2. Verwendung nach Anspruch 1,
dadurch gekennzeichnet,
daß die Aktivierung in überwiegend wäßriger Lösung bei einem pH-Wert zwischen 8 und 11 erfolgt.

3. Verwendung nach Anspruch 1 oder 2,
dadurch gekennzeichnet,
daß in der Lösung als anorganische Peroxoverbindung Natriumperborat oder Natriumpercarbonat vorliegt.

4. Verwendung nach einem oder mehreren der Ansprüche 1 bis 3,
dadurch gekennzeichnet,
daß pro Äquivalent Aktivsauerstoff des Wasserstoffperoxids und/oder der anorganischen Peroxoverbindung 1 bis 0,05 Mol, vorzugsweise 0,5 bis 0,1 Mol eines Aktivators der Formel (I), insbesondere des Aktivators mit R¹ gleich n-Octyl und R²=H, eingesetzt wird.

5. Oxidations-, Bleich-, Reinigungs- oder Desinfektionsmittel, enthaltend eine in Gegenwart von Wasser Wasserstoffperoxid freisetzende anorganische Peroxoverbindung und einen Aktivator auf der Basis einer N-Alkanoylverbindung,
dadurch gekennzeichnet,
daß es als Aktivator eine Verbindung der allgemeinen Formel (I) gemäß Anspruch 1 enthält.

6. Mittel nach Anspruch 5,
dadurch gekennzeichnet,
daß es pro Mol anorganische Peroxoverbindung 1 bis 0,05 Mol, vorzugsweise 0,5 bis 0,1 Mol Aktivator der Formel (I), enthält.

7. Mittel nach Anspruch 5 oder 6,
dadurch gekennzeichnet,
daß es als anorganische Peroxoverbindung Natriumperborat-monohydrat, Natriumperborattetrahydrat, superoxidiertes Natriumperborat oder Natriumpercarbonat enthält.

8. Wasch- und Bleichmittel nach Anspruch 5,
dadurch gekennzeichnet,
daß es im wesentlichen enthält:
2 - 35 Gew.-% anorganische Peroxoverbindung
0,2 - 20 Gew.-% Aktivator der Formel (I)
5 - 30 Gew.-% anionische und/oder nichtionische Tenside
5 - 60 Gew.-% anorganische Gerüstsubstanzen
0 - 20 Gew.-% organische Gerüstsubstanzen
ad 100 Gew.-% übliche Hilfsstoffe und Wasser.

9. Bleichzusatzmittel gemäß Anspruch 5,
dadurch gekennzeichnet,
daß es im wesentlichen enthält:
5 - 50 Gew.-% anorganische Peroxoverbindung
2 - 30 Gew.-% Aktivator der Formel (I)
0 - 5 Gew.-% Peroxid stabilisatoren
0 - 40 Gew.-% pH-regulierende Mittel
ad 100 Gew.-% andere übliche Hilfsstoffe und Wasser.

10. Aktivator der Formel (I) gemäß Anspruch 1.
